(19) Europäisches Patentamt
European Patent Office
Office européen des brevets

(11) Veröffentlichungsnummer: **0 001 060**
**B1**

(12) # EUROPÄISCHE PATENTSCHRIFT

(45) Veröffentlichungstag der Patentschrift: **06.10.82**

(21) Anmeldenummer: **78100684.6**

(22) Anmeldetag: **17.08.78**

(51) Int. Cl.³: **C 07 B 20/00**, C 07 B 19/00, C 07 C 99/10, C 07 C 101/04, C 07 C 101/72, C 07 C 101/78, C 07 C 121/66

(54) **Verfahren zur Herstellung von optisch aktiver, ggf. substituierter 2-Amino-2-phenylessigsäure.**

(30) Priorität: **30.08.77 DE 2738934**

(43) Veröffentlichungstag der Anmeldung: **21.03.79 Patentblatt 79/6**

(45) Bekanntmachung des Hinweises auf die Patenterteilung: **06.10.82 Patentblatt 82/40**

(84) Benannte Vertragsstaaten: **BE CH DE FR GB NL**

(56) Entgegenhaltungen:
**DE-A-2 227 011**
**DE-A-2 309 180**
**FR-A-2 334 658**
**GB-A-1 388 341**
**US-A-3 832 388**
**HOUBEN–WEYL »Methoden der organischen Chemie«, 4. Auflage, Band IV, Teil 2, 1955, Georg Thieme Verlag, Stuttgart, Seiten 511–513**

**Die Akte enthält technische Angaben, die nach dem Eingang der Anmeldung eingereicht wurden und die nicht in dieser Patentschrift enthalten sind.**

(73) Patentinhaber: **RIEDEL-DE HAEN AKTIENGESELLSCHAFT, Wunstorfer Strasse 40, D-3016 Seelze 1 (DE)**

(72) Erfinder: **Schmand, Horst, Dr,, Riepener Strasse 17, D-3052 Bad Nenndorf (DE)**
Erfinder: **Dannenberg, Wolfgang, Dr., Düendorfer Weg 20, D-3050 Wunstorf (DE)**

(74) Vertreter: **Reuter, Johann-Heinrich, Dr. et al, HOECHST AKTIENGESELLSCHAFT** Zentrale Patentabteilung **Postfach 80 03 20, D-6230 Frankfurt/Main 80 (DE)**

**EP 0 001 060 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Verfahren zur Herstellung von optisch aktiver, ggf. substituierter 2-Amino-2-phenylessigsäure

Die Erfindung betrifft ein Verfahren zur Herstellung von optisch aktiver, ggf. substituierter 2-Amino-2-phenylessigsäure durch asymmetrische Transformation eines entsprechenden DL-Aminophenylacetonitrils mit L(+)-Weinsäure.

Nach dem erfindungsgemäßen Verfahren lassen sich optisch aktive Verbindungen der allgemeinen Formel (I) (C-Phenylglycine) herstellen

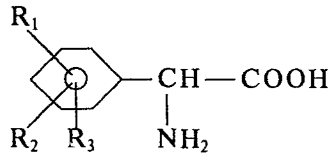

(I)

worin $R_1$, $R_2$ und $R_3$ gleich oder verschieden sind und jeweils ein Wasserstoff- oder Halogenatom oder ein Hydroxy-, Alkyl-, Acyl- oder Alkoxygruppe bedeuten.

Optisch aktive Verbindungen dieses Typs sind bereits bekannt, und stellen u. a. wichtige Vorprodukte für die Herstellung von halbsynthetischen $\beta$-Laktam-Antibiotika, z. B. von Ampicillin, dar.

Zur Herstellung dieser Verbindungen, insbesondere von optisch aktiver 2-Amino-2-phenylessigsäure (Phenylglycin), sind verschiedene Wege eingeschlagen worden:

1. Die direkte optische Trennung:
   Da Phenylglycin als Betain reagiert, wurde versucht, sowohl über die Aminogruppe als auch über die Säuregruppe eine Salzbildung mit einer optisch aktiven Verbindung als Vorbedingung für eine optische Trennung zu erreichen.
   An der Aminogruppe kommt es nur mit sehr starken optisch aktiven Säure, z. B. Campfer (10) sulfonsäure, zu einer Salzbildung. Es wird nur eine Ausbeute von 41% an D(−)-Phenylglycin bezogen auf DL-Phenylglycin erreicht (Ja-OS 76 95.036). Außerdem muß das nicht gewünschte L(+)-Stereomere unter drastischen Bedingungen razemisiert werden.
   Andererseits wurde versucht, die Aminogruppe z. B. durch eine N-Acetyl-Substitution zu blocken und dann eine Salzbildung mit einer optisch aktiven Base zu erreichen. Abgesehen davon, daß auf diesem Weg nur Ausbeuten von 16% D(−)-Phenylglycin bezogen auf DL-Phenylglycin erreicht wurden, fällt der hohe Preis optisch aktiver Basen nachteilig ins Gewicht (CS-PS 121 645 nach CA Referat 68 (1968) 22 254 s).
   Weiterhin sind Versuche bekannt, durch Animpfen einer Salzlösung mit einem Enantiomeren und anschließende selektive Kristallisation zu optisch aktivem Phenylglycin zu kommen. Jedoch kann wegen des geringen Effekts nicht chargenweise gearbeitet werden und die kontinuierlichen Verfahren erfordern komplizierte, mit einem hohen Aufwand an Regeltechnik ausgestattete Apparaturen (DE-OS 2 319 493).
2. Die optische Trennung von Phenylglycinestern mit anschließender Esterverseifung.
   In der DE-OS 2 227 011 wird ein DL-Phenylglycinester mit (+)-Weinsäure in Anwesenheit einer Mischung von Lösungsmitteln verschiedener Polaritäten, von denen eines ein Alkanol mit 1−4 C-Atomen ist, behandelt und das (+)-Hemitartrat des D-Phenylglycinesters selektiv auskristallisiert. Für den Schritt der optischen Trennung werden Ausbeuten von 66−83% angegeben, für den folgenden Schritt der Hydrolyse zum Phenylglycin 60−75%, so daß die Ausbeute vom razemischen Ester zur optisch aktiven Säure 40−60% beträgt.
   In der DE-OS 2 309 180 wird ein Verfahren zur Herstellung eines Esters eines Enantiomeren einer $\alpha$-Aminoessigsäure in Form eines Salzes mit einer optisch aktiven Säure beschrieben, bei dem ein Ester des entgegengesetzten Enantiomeren dieser $\alpha$-Aminosäure mit der genannten optisch aktiven Säure und einem Aldehyd oder Keton umgesetzt wird, wobei ein Ester des gewünschten Enantiomeren in Form des genannten Salzes ausgeschieden wird. So kann man den DL-Ester in Anwesenheit von L(+)-Weinsäure mit Benzaldehyd oder Aceton in Äthanol oder Methanol als Lösungsmittel umsetzen und den D-Phenylglycinester als Hemitartratsalz erhalten. Die erhaltenen Ausbeuten betragen z. T. über 90%. Für die anschließende Stufe der Esterhydrolyse werden gleichfalls 90% Ausbeute angegeben.
   Grundsätzlich ist hinsichtlich beider Verfahren zu berücksichtigen, daß in der Stufe der Hydrolyse die Gefahr der Esterrazemisierung besteht.
   Veresterungen und Esterverseifung sind außerdem zusätzliche Syntheseschritte von der Stufe des synthetisierten DL-C-Phenylglycins ausgehend, die mit Ausbeuteverlusten verbunden sind.
3. Die optische Trennung von 2-Amino-2-phenylacetonitril und anschließende Verseifung des Nitrils.
   Die Racematspaltung von DL-2-Amino-2-phenyl-acetonitril mit L(+)-Weinsäure ist seit 1932 bekannt. H. Reihlen et al. untersuchten in einer Reihe von Arbeiten die Konfiguration von Derivaten der 2-Amino-2-phenylessigsäure. Dabei wurde das diastereomere D(+)-2-Amino-2-

phenylacetonitril-L(+)-hydrogentartrat fraktioniert aus Methanol kristallisiert und eine spezifische Drehung des Salzes von $[\alpha]_D = +41{,}1°$ ($H_2O$) erhalten.
Beim Einengen wurde die Mutterlauge rot und schmierig. Aus den Mengenangaben errechnet sich eine Ausbeute von 32% der Theorie, bezogen auf racemisches Nitril (Liebigs Ann. d. Chemie 534, (1938), 247).
D. G. Neilson und D. F. Ewing führten 1966 diese Arbeiten weiter. Sie spalteten das Aminonitril nach den Angaben von H.Reihlen und verbesserten die spez. Drehung des D-Aminonitril-L(+)-hydrogentartrats auf $[\alpha]_D = +45{,}4°$ (c=0,9; $H_2O$). Es wurde keine Ausbeute angegeben. Die Autoren beobachteten ebenfalls weitgehende Zersetzung bei Umkristallisationsversuchen des Salzes aus Methanol (J. Chem. Soc. C (1966) 393).

Nach dem ungarischen Patent 154 410 wird aus dem Bezolextrakt einer klassischen Strecker-Reaktion mit L(+)-Weinsäure und zusätzlichem Methanol 30% der Theorie rohes Hydrogentartrat ($[\alpha]_D = +41-43°$ (c=3; $H_2O$)) und nach Umkristallisation aus Benzol/Methanol ca. 18% der Theorie, bezogen auf Benzaldehyd, Feinausbeute ($[\alpha]_D^{20} = +44°$ (c=3; $H_2O$)) erhalten. Verseifung dieses Salzes in siedender 20%iger Salzsäure lieferte D(−)-C-Phenylglycin.
In der GB-PS 1 382 687 wird ein Verfahren beschrieben, bei dem racemisches 2-Amino-2-phenylacetonitril mit optisch aktiver Weinsäure in Gegenwart von Alkansäuren mit 2−8 C-Atomen zum Weinsäuresalz eines Enantiomeren obigen Acetonitrils umgesetzt wird.
Aus der GB-PS 1 388 341 ist die anschließende Stufe der Nitrilverseifung bekannt. Bei dem Verfahren wird D(−)-2-Amino-2-(p-hydroxyphenyl)-acetonitril in einem Lösemittel-Gemisch aus Benzol, Ethylacetat und Methanol mit L(+)-Weinsäure umgesetzt, und das isolierte Nitriltartrat wird dann mit Salzsäure verseift. Dabei werden etwa 2,2 l Lösemittel-Gemisch pro Mol $\alpha$-Aminosäurenitril-Racemat verwendet, und die Ausbeute an gewünschtem Salz beträgt nur 28 Prozent der Theorie (bezogen auf L(+)-Weinsäure). Auch Ketone sollen als Lösemittel geeignet sein, ohne daß dies jedoch experimentell belegt wird. Die Besonderheiten des Verfahrens, nämlich der Einsatz einer relativ hohen Lösemittelmenge und die Verfahrensweise (40 Stunden Stehenlassen), sind als Indiz dafür zu werten, daß eine klassische Racematspaltung über fraktionierte Kristallisation der diastereomeren Salze durchgeführt wurde.
Die Verfahren der erwähnten DE-OS 2 227 011 und GB-PS 1 382 687 unterscheiden sich von den übrigen über fraktionierte Kristallisation der Salze verlaufenden Verfahren dadurch, daß mit einem geeigneten Hilfsstoff das Diastereomerenpaar in ein diasteomeres Salz allein überführt wird. Dieser Prozeß, der gleichzeitig Racemisierung und Trennung zu einem optischen Isomeren umschließt, wird asymmetrische Transformation 2. Art genannt (E. E. Turner, Quart. Rev. 1947/1, 299 ff.).
Die Nachteile der aufgeführten Verfahren des Standes der Technik bestehen in den geringen Ausbeuten bzw. in der Verwendung von zum Teil sehr teuren, zum Teil aus Gründen der Geruchsbelästigung nur mit erhöhtem Aufwand zu handhabenden Hilfschemikalien.
Der Erfindung liegt die Aufgabe zugrunde, ein Verfahren zur Herstellung optisch aktiver 2-Aminophenyl-essigsäure bereitzustellen, bei dem unter Verwendung billiger, einfach zu handhabender Hilfschemikalien und bei einem einfachen Verfahrensablauf eine hohe Endausbeute erreicht wird.
Überraschenderweise wurde nunmehr gefunden, daß Alkanole und/oder Ketone das DL-Aminophenylacetonitril-hydrogentartratsalz einer asymmetrischen Transformation unterwerfen. Dies ist umso erstaunlicher, als bei den beschriebenen früheren Versuchen zur Diastereomerentrennung des Salzes in Methanol weitgehend Zersetzung beobachtet wurde.
Die Erfindung betrifft ein Verfahren zur Herstellung von optisch aktiver 2-Amino-2-phenylessigsäure der allgemeinen Formel (I)

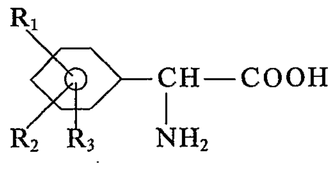

(I)

worin $R_1$, $R_2$ und $R_3$ gleich oder verschieden sind und jeweils ein Wasserstoff- oder Halogenatom oder eine Hydroxy-, Alkyl-, Acyl- oder Alkoxygruppe bedeuten, durch (a) Umsetzung eines DL-2-Amino-2-phenylacetonitrils der allgemeinen Formel (II)

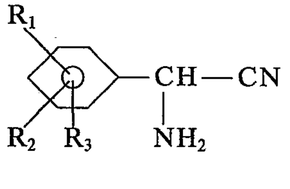

(II)

worin $R_1$, $R_2$ und $R_3$ die oben angegebene Bedeutung haben, mit L-(+)-Weinsäure in Gegenwart mindestens eines Alkanols mit 1 bis 5 C-Atomen und/oder mindestens eines Ketons aus der Gruppe der Dialkylketone, cycloaliphatischen Ketone oder Arylalkylketone, gegebenenfalls im Gemisch mit einem inerten Verdünnungsmittel aus der Stoffklasse der aromatischen Kohlenwasserstoffe, halogenierten aliphatischen Kohlenwasserstoffe, Ester oder Äther, bei einer Temperatur von 0 bis 50°C, (b) Belassen des Umsetzungsproduktes bei dieser Temperatur über einen Zeitraum von 2 bis 120 Stunden, (c) Abtrennung des auskristallisierten Transformationsproduktes, (d) Verseifung durch Behandeln mit Säure und (e) Isolierung der entstandenen optisch aktiven 2-Amino-2-phenylessigsäure, das dadurch gekennzeichnet ist, daß die (L)-(+)-Weinsäure in äquimolarer Menge und das Alkanol und/oder das Keton oder gegebenenfalls das Gemisch aus Alkanol oder Keton und inertem Verdünnungsmittel in einer Menge von 0,2 bis 1 Liter pro Mol DL-2-Amino-2-phenylacetonitril der Formel II verwendt werden und die Transformation gemäß Stufe b) unter Rühren durchgeführt wird.

Vorzugsweise werden die niederen Alkohole im Gemisch untereinander eingesetzt wie Methanol/Äthanol oder mit Lösungsmitteln aus folgenden Stoffklassen verdünnt: mit aromatischen Kohlenwasserstoffen, aliphatischen Kohlenwasserstoffen, chlorierten Kohlenwasserstoffen, Äthern und Estern. Ferner kann man vorteilhaft Alkanol/Ketongemische zur Transformation einsetzen. Die niederen Ketone wie Aceton oder Methyläthylketon lassen sich jedoch auch allein einsetzen.

Die Transformation mit Alkanolen erfährt bei Raumtemperatur eine deutliche Beschleunigung bei Gegenwart von Ketonen, z. B. Aceton, Methyläthylketon oder Acetophenon. Die Konzentration des Ketons kann dabei von 1 Vol.-% bis über 50 Vol.-% variiert werden. Bevorzugt werden äquimolare Mengen Keton angewandt.

Zur Erzielung einer günstigen Ausbeute (>50% der Theorie bezogen auf DL-α-Aminonitril-Derivat) sind die verwendeten Lösemittelmengen möglichst gering zu halten. Die Suspension des α-Aminonitril-hemitartratsalzes muß jedoch rührfähig sein, damit die Transformation gleichmäßig und vollständig erfolgt.

Je nach Lösemittel bzw. Zusammensetzung des Lösemittelgemisches, bestehend aus Alkanol und/oder Keton, aus Alkanol und inertem Verdünnungsmittel, aus Keton und inertem Verdünnungsmittel oder auch aus Alkanol, Keton und inertem Verdünnungsmittel, werden für die Umsetzung pro Mol DL-2-Amino-2-phenylacetonitril der allgemeinen Formel (II) 0,2–1 l, bevorzugt 0,4–0,5 l des oben angegebenen Lösemittels bzw. Lösemittelgemisches verwendet.

Die Transformation wird bei 0–50°C innerhalb von 2–120 h durchgeführt. Dabei ist im Gegensatz zur Transformation in Alkancarbonsäuren kein unbedingter Ausschluß von Wasser erforderlich. Die genauen Bedingungen sind im Einzelfall empirisch durch Variation von Temperatur, Zeit, Zusammensetzung des Lösemittelgemisches und Salzkonzentration zu ermitteln.

Im allgemeinen wird L(+)-Weinsäure in den betreffenden organischen Lösemitteln bei Raumtemperatur (20°C) suspendiert oder gelöst, und das Aminonitril wird fest eingetragen. Man rührt je nach Lösemittel oder Lösemittelgemisch und Temperaturwahl innerhalb der oben angegebenen Zeitspanne im geschlossenen Gefäß, kühlt gegebenenfalls ab und filtriert. Das Salz wird mit einem Lösemittelgemisch wie es bereits zur Transformation verwendet wurde oder mit einem Verdünnungsmittel gewaschen, getrocknet und nach an sich bekannten Verfahren verseift.

Die Weinsäure kann äquimolar aber auch in geringeren als stöchiometrischen Mengen eingesetzt werden. Das substituierte Aminonitril läßt sich auch in gelöster Form dosieren.

Eine besondere Ausführungsform der Erfindung ergibt sich, wenn man das DL-Aminonitril-L-hydrogentartratsalz in Methanol präpariert und anschließend das Lösungsmittel abgedampft wird. Setzt man dieses vorbehandelte Salz, das eine spezifische Drehung von $[\alpha]_D$ 19–23° ($H_2O$) besitzt, zur Transformation in höheren Alkanolen bzw. Ketonen ein, so werden die Reaktionszeiten verkürzt.

Die Transformationen in den erfindungsgemäßen Lösungsmitteln bzw. Gemischen führen ausschließlich zu ggf. substituierten D(+)-2-Amino-2-phenylacetonitril-L(+)-hydrogentartraten, die nach Verseifung in Mineralsäuren, bevorzugt in 20%iger Salzsäure D(–)-Phenylglycine ergeben.

Die Erfindung wird durch folgende Beispiele verdeutlicht, ohne daß dadurch die Allgemeingültigkeit des Erfindungsgedankens beschränkt werden soll:

## Beispiel 1

Zu einer Lösung von 15,0 g (0,1 Mol) L(+)-Weinsäure in 50 ml Methanol gibt man unter Rühren 13,2 g (0,1 Mol) festes DL-2-Amino-2-phenylacetonitril. Die entstehende Suspension wird 8 h bei 30°C gerührt und anschließend läßt man ebenfalls unter Rühren weitere 14 h von 30°C auf ca. 20°C abkühlen.

Zum Schluß wird nochmals 2 h auf 10°C abgekühlt, abgesaugt und mit 20 ml Methanol gewaschen. Man trocknet das Salz bei ca. 50°C und erhält 22,6 g (0,08 Mol) D(+)-Aminophenylacetonitril-L(+)-hydrogentartrat. Das entspricht einer Ausbeute von 80% der Theorie, bezogen auf DL-Aminophenylacetonitril.

$[\alpha]_D^{20} = +44{,}1°$ (c=2; $H_2O$).

Beispiel 2

Zu einer Suspension von 15,0 g (0,1 Mol) L(+)-Weinsäure in einem Lösungsmittelgemisch aus 25 ml Methanol und 20 ml Dichloräthan fügt man unter gutem Rühren 12,2 g (0,1 Mol) DL-2-Amino-2-phenylacetonitril hinzu. Die Suspension wird 24 h bei Raumtemperatur gerührt. Man saugt ab, wäscht mit 25 ml Methanol/Dichloräthan (1 : 1) und trocknet das Salz bei ca. 50° C. Die Ausbeute beträgt 23,8 g (0,0844 Mol) D(+)-Aminophenylacetonitril-L(+)-hydrogentartrat, das entspricht 84,4% der Theorie, bezogen auf DL-Aminophenylacetonitril.

$[\alpha]_D^{20}$ = +43,5° (c=2; $H_2O$).

Beispiel 3

Zu 15 g (0,1 Mol) L(+)-Weinsäure in 60 ml abs. Äthanol gibt man unter Rühren 13,2 g festes (0,1 Mol) DL-2-Amino-2-phenylacetonitril. Die entstehende Suspension wird 52 Stunden lang bei ca. 20° C gerührt. Der Niederschlag wird abgesaugt und mit 30 ml Toluol gewaschen. Man trocknet bei ca. 50° C und erhält 23 g (0,0816 Mol) D(+)-2-Amino-2-phenylacetonitril-L-(+)-hydrogentartrat. Die Ausbeute beträgt 81,6% der Theorie, bezogen auf racemisches Aminonitril.

$[\alpha]_D^{20}$ = +43° (c=2; $H_2O$).

Beispiel 4

150 g (1 Mol) L(+)-Weinsäure werden in einem Lösungsmittelgemisch, bestehend aus 300 ml Äthanol und 90 ml Methyläthylketon suspendiert. Anschließend versetzt man mit 132 g (1 Mol) DL-2-Amino-2-phenylacetonitril und rührt bei Raumtemperatur 23 Stunden lang. Die Suspension des D-Aminonitril-L-hydrogentartratsalzes wird abgesaugt, mit 50 ml Äthanol/Methyläthylketon gewaschen (1 : 2 V/V) und bei 50° C getrocknet. Man erhält 243 g (0,862 Mol) des L(+)-Hydrogentartrats vom D(+)-Amino-phenylacetonitril. Die Ausbeute beträgt 86,2% der Theorie bezogen auf DL-2-Amino-2-phenylacetonitril.

$[\alpha]_D^{20}$ = +44,5° (c=2; $H_2O$).

Beispiel 5

Eine Suspension von 28,2 g (0,1 Mol) DL-Amino-phenylacetonitril-L(+)-hydrogentartrat ($[\alpha]_D$)+19,9° ($H_2O$) wird in 80 ml n-Propanol 120 Stunden lang bei 20° C gerührt.

Man erhält 24,6 g (0,872 Mol) des L-Hydrogentartratsalzes mit D(+)-Aminophenylacetonitril.

Die Ausbeute beträgt 87,2% der Theorie bezogen auf racemisches Aminonitril.

$[\alpha]_D^{20}$ = +43,6° (c=2; $H_2O$).

Beispiel 6

Zu einer Lösung von 8,0 g (0,05 Mol) DL-2-Amino-2-(p-methoxyphenyl)-acetonitril in 40 ml Methyläthylketon werden unter Rühren 7,4 g (0,05 Mol) L(+)-Weinsäure hinzugefügt. Die Suspension wird bei Raumtemperatur 72 Stunden lang gerührt. Anschließend wird das Kristallisat abgetrennt, mit 5 ml Methanol/Toluol (1 : 2) gewaschen und bei 50° C getrocknet. Die Ausbeute beträgt 14,65 g (95,1% der Theorie) D(+)-2-Amino-2-(p-methoxyphenyl)-acetonitril-L(+)-hemitartrat mit einer spezifischen Drehung von $[\alpha]_D^{20}$ = +45° (c=1; $H_2O$).

Beispiel 7

Ein nach Beispiel 1–4 gewonnenes D(+)-2-Amino-2-phenylacetonitril-L(+)-hydrogentartrat ($[\alpha]_D^{20}$ = +43° (c=2; $H_2O$)) wird in 20%iger wäßriger Salzsäure verseift:

90,9 g D(+)-Aminophenylacetonitril-L(+)-hydrogentartrat (0,322 Mol) werden in 376 g siedende 20%ige Salzsäure eingetragen und 45 min am Rückfluß gehalten. Anschließend rührt man bei 80° C 0,5 Stunden lang mit 2 g Aktivkohle aus und filtriert die Lösung oberhalb 50° C ab. Das Filtrat wird bei 50° C mit ca. 160 g 50%iger Natronlauge unter Außenkühlung mit Eis auf pH 7 gestellt. Nach 0,5 Stunden wird der Niederschlag bei ca. 10° C abgesaugt und mit ca. 250 ml Permutitwasser gewaschen.

Zum Schluß wäscht man mit 50 ml Methanol nach und erhält ein farbloses C-Phenylglycin. Es wird bei 50° C getrocknet.

Die Ausbeute beträgt 41,2 g D(–)-2-Amino-2-phenylessigsäure (=84,7% der Theorie), bezogen auf

D(+)-2-Amino-2-phenylacetonitril-L(+)-hydrogentartrat.
Fp.: 307° C subl.; 310–312° C.
$[\alpha]_D^{20} = -146{,}2°$ (c=2; 1n HCl).

**Patentansprüche**

1. Verfahren zur Herstellung von optisch aktiver 2-Amino-2-phenylessigsäure der allgemeinen Formel I

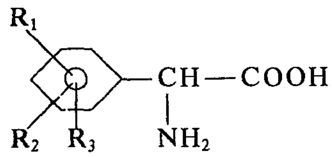

(I)

worin $R_1$, $R_2$ und $R_3$ gleich oder verschieden sind und jeweils ein Wasserstoff- oder Halogenatom oder eine Hydroxy-, Alkyl-, Acyl- oder Alkoxygruppe bedeuten, durch (a) Umsetzung eines DL-2-Amino-2-phenylacetonitrils der allgemeinen Formel II

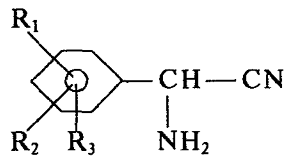

(II)

worin $R_1$, $R_2$ und $R_3$ die oben angegebene Bedeutung haben, mit L-(+)-Weinsäure in Gegenwart mindestens eines Alkanols mit 1 bis 5 C-Atomen und/oder mindestens eines Ketons aus der Gruppe der Dialkylketone, cycloaliphatischen Ketone oder Arylalkylketone, gegebenenfalls im Gemisch mit einem inerten Verdünnungsmittel aus der Stoffklasse der aromatischen Kohlenwasserstoffe, halogenierten aliphatischen Kohlenwasserstoffe, Ester oder Äther, bei einer Temperatur von 0 bis 50° C, (b) Belassen des Umsetzungsproduktes bei dieser Temperatur über einen Zeitraum von 2 bis 120 Stunden, (c) Abtrennung des auskristallisierten Transformationsproduktes, (d) Verseifung durch Behandeln mit Säure und (e) Isolierung der entstandenen optisch aktiven 2-Amino-2-phenylessigsäure, dadurch gekennzeichnet, daß die L-(+)-Weinsäure in äquimolarer Menge und das Alkanol und/oder das Keton oder gegebenenfalls das Gemisch aus Alkanol oder Keton und inertem Verdünnungsmittel in einer Menge von 0,2 bis 1 Liter pro Mol DL-2-Amino-2-phenylacetonitril der Formel II verwendet werden und die Transformation gemäß Sufe b) unter Rühren durchgeführt wird.

2. Verfahren nach Anspruch 1, dadurch gekennzeichnet, daß als Ausgangsmaterial DL-2-Amino-2-phenylacetonitril oder DL-2-Amino-2-(4-methoxyphenyl)-acetonitril verwendet wird.

3. Verfahren nach Anspruch 1, dadurch gekennzeichnet, daß die Umsetzung in Gegenwart eines Gemisches aus einem Alkanol und einem Keton durchgeführt wird, wobei die Konzentration des Ketons 1 bis 50 Volumenprozent des Gemisches beträgt.

4. Verfahren nach Anspruch 1, dadurch gekennzeichnet, daß das Alkanol und/oder das Keton oder das Gemisch aus Alkanol oder Keton und inertem Verdünnungsmittel in einer Menge von 0,4 bis 0,5 Liter pro Mol DL-2-Amino-2-phenylacetonitril der Formel II verwendet wird.

5. Verfahren nach Anspruch 1, dadurch gekennzeichnet, daß gemäß Stufe a) Methanol als Alkanol eingesetzt und abgedampft wird und gemäß Stufe b) ein höheres Alkanol aus der Gruppe der Alkanole mit 1 bis 5 C-Atomen oder ein Keton aus der in Anspruch 1 genannten Gruppe eingesetzt wird.

6. Verfahren nach Anspruch 1, dadurch gekennzeichnet, daß als Keton Aceton, Methyläthylketon oder Acetophenon verwendet wird.

**Claims**

1. Process for the manufacture of optically active 2-amino-2-phenylacetic acid of the general formula I

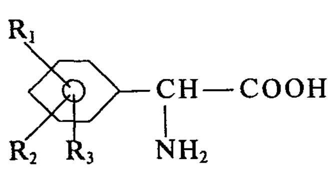

(I)

in which $R_1$, $R_2$ and $R_3$, which are identical or different, each denote a hydrogen or halogen atom or a

hydroxy, alkyl, acyl or alkoxy group, by (a) reacting a DL-2-amino-2-phenyl-acetonitrile of the general formula II

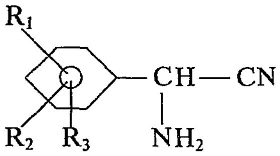

(II)

in which $R_1$, $R_2$ and $R_3$ have the above meaning, with L(+)-tartaric acid in the presence of at least one alkanol having from 1 to 5 carbon atoms and/or at least one ketone from the group of dialkyl ketones, cycloaliphatic ketones or arylalkyl ketones, optionally in admixture with an inert diluent from the class of compounds consisting of aromatic hydrocarbons, halogenated aliphatic hydrocarbons, esters or ethers, at a temperature of from 0 to 50° C, (b) leaving the reaction product at this temperature for 2 to 120 hours, (c) separating the crystallized transformation product, (d) hydrolyzing same by a treatment which an acid and (e) isolating the optically active 2-amino-2-phenylacetic acid formed, characterized in that the L-(+)-tartaric acid is used in an equimolar amount, and the alkanol and/or the ketone or optionally the mixture of alkanol or ketone and inert diluent is used in an amount of 0,2 to 1 litre per mole of DL-2-amino-2-phenyl-acetonitrile of the formula II, and the transformation according to step (b) is performed by stirring.

2. Process according to claim 1, characterized in that as starting material there is used DL-2-amino-2-phenylacetonitrile or DL-2-amino-2-(4-methoxyphenyl)-acetonitrile.

3. Process according to claim 1, characterized in that the reaction is carried out in the presence of a mixture of an alkanol and a ketone, the concentration of the ketone being 1 to 50 per cent by volume of the mixture.

4. Process according to claim 1, characterized in that the alkanol and/or the ketone or the mixture of alkanol or ketone and inert diluent is used in an amount of from 0.4 to 0.5 litre per mole of DL-2-amino-2-phenylacetonitrile of the formula II.

5. Process according to claim 1, characterized in that, according to step (a), methanol is used as alkanol and evaporated and, according to step (b), a higher alkanol from the group of alkanols having 1 to 5 carbon atoms or a ketone from the group mentioned in claim 1 is used.

6. Process according to claim 1, characterized in that as ketone there is used acetone, methyl ethyl ketone or acetophenone.

**Revendications**

1. Procédé de préparation d'un acide amino-2 phényl-2 acétique optiquement actif qui répond à la formule g+nérale (I):

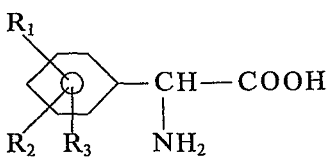

(I)

dans laquelle $R_1$, $R_2$ et $R_3$ sont identiques ou différents et représentent chacun un atome d'hydrogène ou d'halogène ou un radical hydroxy, alkyle, acyle ou alcoxy, par (a) réaction d' un amino-2 phényl-2 acétonitrile-(DL) répondant à la formule générale (II):

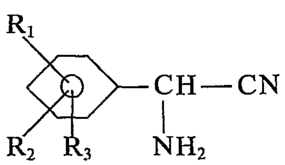

(II)

dans laquelle $R_1$, $R_2$ et $R_3$ ont les significations précédemment données, avec l'acide tartrique-(L) (+) en présence d'au moins un alcanol contenant de 1 à 5 atomes de carbone et/ou d'au moins une cétone prise dans l'ensemble constitué par les dialkylcétones, les cétones cycloaliphatiques et les aryl-alkyl-cétones, éventuellement en mélange avec un diluant inerte pris dans l'ensemble constitué par les hydrocarbures aromatiques, les hydrocarbures aliphatiques halogénés, les esters et les éthers, à une température de 0 à 50°C, (b) abandon du produit réactionnel à cette température pendant 2 à 120 heures, (c) séparation du produit de transformation cristallisé, (d) saponification par traitement avec un acide et (e) isolement de l'acide amino-2 phényl-2 acétique optiquement actif qui a été ainsi formé, procédé caractérisé en ce que l'acide tartrique-(L) (+) est utilisé en une quantité équimolaire et

l'alcanol et/ou la cétone, ou éventuellement le mélange de l'alcanol ou de la cétone et d'un diluant inerte, sont utilisés en une quantité de 0,2 à 1 litre par mole d'amino-2 phényl-2 acétonitrile-(DL) de formule (II), et la transformation de l'étape (b) est effectuée sous agitation.

2. Procédé selon la revendication 1, caractérisé en ce qu'on utilise, comme corps de départ, l'amino-2 phényl-2 acétonitrile-(DL) ou l'amino-2 (méthoxy-4 phényl)-2 acétonitrile-(DL).

3. Procédé selon la revendication 1, caractérisé en ce qu'on effectue la réaction en présence d'un mélange d'un alcanol et d'une cétone, la concentration de la cétone étant de 1 à 50% en volume par rapport au mélange.

4. Procédé selon la revendication 1, caractérisé en ce que l'alcanol et/ou la cétone ou le mélange d'un alcanol ou d'une cétone et d'un diluant inerte sont utilisés en une quantité de 0,4 à 0,5 litre par mole d'amino-2 phényl-2 acétonitrile-(DL) de formule (II).

5. Procédé selon la revendication 1, caractérisé en ce que, dans l'étape (a), on utilise le méthanol comme alcanol et on évapore, et, dans l'étape (b), on utilise un alcanol supérieur pris dans l'ensemble des alcanols contenant de 1 à 5 atomes de carbone ou une cétone prise dans l'ensemble défini à la revendication 1.

6. Procédé selon la revendication 1, caractérisé en ce qu'on utilise, comme cétone, l'acétone, la méthyl-éthylcétone ou l'acétophénone.